(19) Europäisches Patentamt
European Patent Office
Office européen des brevets

(11) Publication number: 0 000 153
A1

(12) # EUROPEAN PATENT APPLICATION

(21) Application number: 78100168.0

(22) Date of filing: 15.06.78

(51) Int. Cl.[2]: C 07 D 215/56, A 61 K 31/47

(30) Priority: 20.06.77 US 807914

(43) Date of publication of application: 10.01.79 Bulletin 79/1

(84) Designated Contracting States: BE CH DE FR GB LU NL SE

(71) Applicant: SANDOZ LTD.,
Lichtstrasse 35,
CH-4002 Basel (CH)

(72) Inventor: Hardtmann, Goetz Eduard,
12 Lynnfield Drive,
Morristown, N.J. 07960 (US)

(54) **4-Hydroxy-2-quinolinone-3-carboxylic acids, their preparation and pharmaceutical compositions containing them.**

(57) Pharmaceutical compositions comprising 4-hydroxy-2-quinolinone-3- carboxylic acids and their salts, and certain compounds of this class, for example 1-allyl-6,7-dimethoxy-4-hydroxy -2-quinolinone-3- carboxylic acid, of the formula

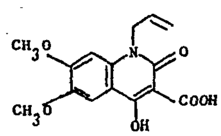

They may be prepared by hydrolysis of the corresponding alkyl esters.

The compounds are useful in the prophylactic treatment of allergic asthma.

EP 0 000 153 A1

Croydon Printing Company Ltd.

TITLE MODIFIED
see front page

IMPROVEMENTS IN OR RELATING TO ORGANIC COMPOUNDS

This invention relates to 4-hydroxy-2-quinolinone-3-carboxylic acids and their salts.

The invention provides pharmaceutical compositions comprising compounds of formula Ip

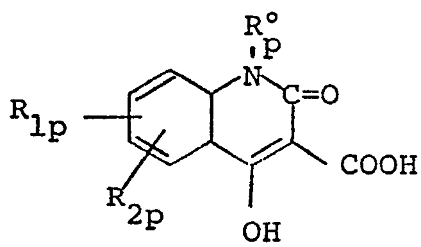

$I_p$

in which $R_p^o$ is hydrogen, alkyl of 1 to 6 carbon atoms, cycloalkyl of 3 to 6 carbon atoms, cycloalkylalkyl in which the cycloalkyl part is of 3 to 6 carbon atoms and the alkyl part is of 1 or 2 carbon atoms, alkenyl or alkynyl of 3 to 6 carbon atoms in which the unsaturation is on other than the alpha carbon atom, or

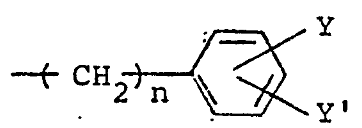

,

in which n is 0 or 1 and

Y and Y' are independently hydrogen, fluorine, chlorine, bromine, alkyl of 1 to 3 carbon atoms, alkoxy of 1 to 3 carbon atoms, trifluoromethyl or nitro with the proviso that only one of Y and Y' can be from the group consisting of nitro and trifluoromethyl and

$R_{1p}$ and $R_{2p}$ are independently hydrogen, fluorine, chlorine, bromine, alkyl of 1 to 4 carbon atoms, alkoxy of 1 to 4 carbon atoms, nitro or trifluoromethyl, with the proviso that only one of $R_{1p}$ and $R_{2p}$ can be from the group consisting of nitro and trifluoromethyl, or $R_{1p}$ and $R_{2p}$ together form a 6,7-methylenedioxy group.

The compounds of formula $I_p$ possess pharmacological activity. In particular they possess disodium chromoglycate (DSCG)-like actiivity, in particular histamine release inhibiting activity, and are therefore indicated for use in the prophylactic treatment of allergic conditions, such as allergic asthma, as indicated in the passive cutaneous anaphylaxis test in the rat. Female rats (180-200 g) are sensitised

by subcutaneous administration of 1 mg of egg albumin (Merck Nr. 967) and 200 mg $Al(OH)_3$ in 1 ml of physiological saline and 0.5 ml of Haemophiluspertussis vaccine (Schweizerisches Serum and Impfinstitut, Bern; Nr. 115 325; $4 \times 10^{10}$ organism/ml) intraperitoneally. Fourteen days later, the animals are exsanguinated, the blood centrifuged, the serum collected and deep frozen. The serum thus obtained (anti-serum) is injected intradermally (0.1 ml of 1:200 diluted serum per injection site) at four sites on the backs of untreated, female rats. Twenty-four hours later each rat is administered 0.1 to 5.6 mg/kg i.v. or 0.1 to 100 mg/kg p.o. of the test compound, and either immediately or 5 to 30 minutes afterwards, in the case of intravenous administration, or 15 or 60 minutes afterwards, in the case of oral administration, egg albumin (5 mg/ml i.v.) dissolved in physiological saline containing 0.25% Evans Blue dye (Merck Nr. 3169). The egg albumin elicits a cutaneous anaphylatic reaction, the intensity of which is proportional to the extent to which the Evans Blue dye diffuses into the tissue surrounding each of the four sensitisation sites. Thirty minutes after the administration of the egg albumin, the rats are killed with ether, the underside of the skin of the back of each animal is exposed and the diameter of the areas of blue dye surrounding each of the four sensitisation sites are measured. Each dose of test compound is investigated in between four and six

rats and the mean diameter compared with the mean value obtained in four solvent-treated control rats. The percentage inhibition is taken as the percentage of the mean diameter in the test animals relative to the mean diameter in the controls.

The DSCG-like activity, in particular histamine release inhibiting activity, can be confirmed by inhibition of histamine release in the rat peritoneal mast cell test, basically as described by Kusner et al., J. Pharmacol. Exp. Therap. 184, 41-46 (1973), with the following modification: after sedimentation of the mast cells by centrifugation at 350 x g and 4°C, the sediments are taken up in 1 ml of Hank's balanced salt solution (HBSS) (buffered to a pH of 6.9) and pooled. The resulting suspension is centrifuged, washed again with HBSS and sedimented. The thus purified mast cells are prepared as 2 ml suspensions in HBSS. To these are added either 2 ml of HBSS, to determine the spontaneous histamine release, or 2 ml of HBSS and 2.24 µg of compound 48/80 (N-methyl-homoanisylamineformaldehyde condensate; a histamine liberator from Burroughs Wellcome and Co. Inc., Tuckahoe, N.Y. USA), to determine the 48/80 induced histamine release, or 2 ml of HBSS with 2.24 µg of 48/80 and from 18 to 180 µg/ml of the test compound, to determine the 48/80 induced histamine release in the presence of the test compound.

The 48/80 induced histamine release minus the spontaneous histamine release is taken as 100% histamine release. The 48/80 induced histamine release in the presence of the test compound minus the spontaneous histamine release is then compared with the 100% value to determine the percentage inhibition by the test compound. [The histamine determination is effected in conventional manner, for example as described in the above-mentioned Kusner et al. article, or in Kusner and Herzig, Advances in Automated Analysis, 429 (1971).] An indicated suitable daily dosage is from 20 to 800 mg preferably administered in divided dosages of from 5 to 400 mg 2 to 4 times a day, or in retard form.

The compounds may be used in free acid form or in the form of their pharmaceutically acceptable mono- or di-base salts, which salt forms have the same order of activity as the free acid forms. Suitable salts include the sodium, potassium and lithium mono- and disalts.

The compounds of formula $I_p$ may be admixed with conventional pharmaceutically acceptable diluents or carriers and, optionally, other excipients, and administered in such forms as tablets or capsules.

The compound 1-methyl-4-hydroxy-2-quinoline-3-carboxylic acid has been described in Mitscher et al., "Heterocycles" Vol. 3, No. 11, pp 913-919 (1975), but to our knowledge no useful pharmacological activity has been

given for this compound.

Accordingly, the invention further provides novel compounds within the scope of formula $I_p$ above. Such novel compounds are those of formula I

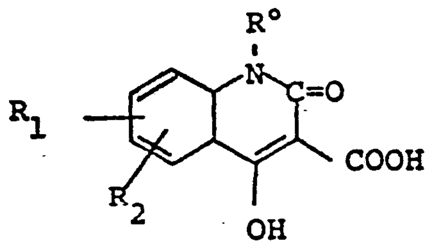

I

in which either

a) R° is cycloalkyl of 3 to 6 carbon atoms, cycloalkylalkyl in which the cycloalkyl part is of 3 to 6 carbon atoms and the alkyl part is of 1 or 2 carbon atoms, alkenyl or alkynyl of 3 to 6 carbon atoms in which the unsaturation is on other than the alpha carbon atom, or

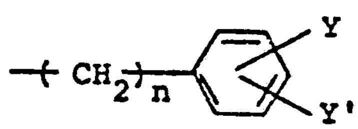

in which n, Y and Y' are as defined above, and $R_1$ and $R_2$ have the same significance as $R_{1p}$ and $R_{2p}$ above, provided that $R_1$ and $R_2$ do not together form a 6,7-methylenedioxy group,

b) R° is hydrogen, $R_1$ is hydrogen, fluorine,

chlorine, bromine, alkyl of 1 to 4 carbon atoms or alkoxy of 1 to 4 carbon atoms and $R_2$ is fluorine, chlorine, bromine, alkyl of 1 to 4 carbon atoms, alkoxy of 1 to 4 carbon atoms, nitro or trifluoromethyl,

c) $R^o$ is alkyl of 1 to 6 carbon atoms and $R_1$ and $R_2$ are independently alkoxy of 1 to 4 carbon atoms,

or d) $R^o$ has the same significance as $R^o_p$ above, and $R_1$ and $R_2$ together form a 6,7-methylenedioxy group.

Preferred significances of $R^o$ are hydrogen, alkyl or alkenyl, more preferably alkyl or alkenyl, particularly allyl.Preferred significances of $R_1$ and $R_2$ are dialkoxy, more preferably 6,7-dialkoxy, particularly 6,7-dimethoxy. Combinations of these preferred significances are especially advantageous.

Preferably the compounds of formula I are in the free acid or disalt form, more preferably the free acid form.

The invention also provides a process for the preparation of compounds of formula I, characterised by hydrolysing a compound of formula II

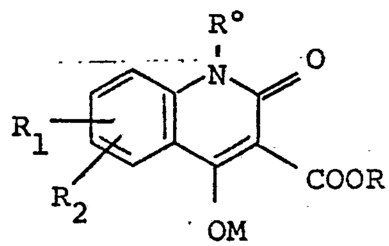

II

in which R°, $R_1$ and $R_2$ are as defined above,

R is alkyl of 1 to 6 carbon atoms,

and M is hydrogen or a pharmaceutically acceptable cation.

When it is desired to obtain the compound of formula I in the disalt form, a suitable procedure is alkaline hydrolysis of the compound of formula II in an aqueous medium at a temperature of 40-150°C, preferably 80-120°C, followed by recovery under conditions avoiding acidification, which can lead to decarboxylation of the product. Preferably M is hydrogen and at least 2 mole equivalents of base are used, preferably sodium or potassium hydroxide to give the disodium or dipotassium salt. When M is itself a cation, mixed disalts may be prepared.

When it it desired to obtain the free acid form of compounds of formula I, a suitable procedure is mild acid hydrolysis of a compound of formula II in which R is an acid-labile group such as t-butyl. The temperature is suitably from -20° to 60°C, preferably from -10° to 35°C in order to avoid decarboxylation of the product. Suitable acid catalysts are sulphuric acid, hydrochloric

acid and perchloric acid, preferably the latter. The reaction medium is suitably a water-miscible non-hydroxylic organic solvent, for example acetonitrile.

Compounds of formula I in free acid form may be treated with base in conventional manner to obtain the monosalt form, in which the salt-forming cation is associated with both the 4-hydroxy and the 3-carboxyl functions, and the disalt form.

The same process, with appropriate starting materials, may be used to prepare the other compounds of formula $I_p$.

The compounds of formula II are described and claimed in our German DOS 2 706 752, and may be prepared by the methods disclosed therein.

The following Examples illustrate the invention:

EXAMPLE 1: 1-Allyl-6,7-dimethoxy-4-hydroxy-2-quinolinone-3-carboxylic acid

A: 1-Allyl-6,7-dimethoxy-4-hydroxy-2-quinolinone-3-carboxylic acid t-butyl ester

To a solution of 8.3 g of di-t-butyl malonate in 75 ml of dimethylacetamide is added portionwise 1.9 g of pentane-washed 50% sodium hydride. The resulting solution is stirred at room temperature for 30 minutes and there is then added 10.0 g of 1-allyl-6,7-dimethoxyisatoic anhydride in 100 ml of dimethylacetamide. The resulting solution is heated at 120°C for 3 hours, the dimethylacetamide removed *in vacuo*, water added, the resulting mixture washed with methylene chloride, acidified with 2 N. hydrochloric acid and extracted with methylene chloride. The organic phase is dried and evaporated *in vacuo* to an oil which is crystallized from ether to obtain 1-allyl-6,7-dimethoxy-4-hydroxy-2-quinolinone-3-carboxylic acid t-butyl ester.

B: 1-Allyl-6,7-dimethoxy-4-hydroxy-2-quinolinone-3-carboxylic acid

To a solution of 3.1 g of 1-allyl-6,7-dimethoxy-4-hydroxy-2-quinolinone-3-carboxylic acid t-butyl ester in 30 ml of acetonitrile at 0°C is added 0.75 ml of 60% aqueous perchloric acid. The resulting precipitate is recovered by filtering and washed with ether to obtain

1-allyl-6,7-dimethoxy-4-hydroxy-2-quinolinone-3-carboxylic acid which melts at 169-170°C and decarboxylates at 185-195°C.

EXAMPLES 2-26:

In manner analogous to Example 1, employing appropriate starting materials in approximately equivalent amounts, the compounds of formula I listed in Table I below may be obtained.

Table I

| Example No. | R° | $R_1$ | $R_2$ | m.p. |
|---|---|---|---|---|
| 2 | $n-C_4H_9$ | 7-Cl | H | |
| 3 | $CH_3$ | $6-CH_3O$ | H | |
| 4 | $CH_3$ | 6-Cl | H | |
| 5 | H | $6-CH_3$ | H | |
| 6 | allyl | H | H | |
| 7 | (3-butenyl) | $6-CH_3O$ | $7-CH_3O$ | |
| 8 | H | $6-CH_3O$ | $7-CH_3O$ | |
| 9 | H | 6-Cl | H | |
| 10 | cyclopentyl | H | H | |
| 11 | cyclopropyl-methyl | H | H | |
| 12 | o-nitrobenzyl | H | H | |
| 13 | propargyl | H | H | |
| 14 | p-fluorobenzyl | H | H | |
| 15 | phenyl | H | H | |
| 16 | methyl | $6-CH_3O$ | $7-CH_3O$ | 216-219° |
| 17 | 2-butynyl | $6-CH_3O$ | $7-CH_3O$ | |
| 18 | 2-methyl-3-propenyl | $6-CH_3O$ | $7-CH_3O$ | |
| 19 | propyl | $6-CH_3O$ | $7-CH_3O$ | |
| 20 | cyclopropyl-methyl | $6-CH_3O$ | $7-CH_3O$ | |
| 21 | 2-butenyl | $6-CH_3O$ | $7-CH_3O$ | |
| 22 | allyl | 6,7-methylenedioxy | | |

Table I (cont'd)

| Example No. | R° | $R_1$ | $R_2$ | m.p. |
|---|---|---|---|---|
| 23 | allyl | 6-Cl | 7-Cl | |
| 24 | allyl | $6-CH_3O$ | $7-C_2H_5$ | |
| 25 | allyl | $6-CH_3O$ | $7-CH_3$ | |
| 26 | allyl | $6-CH_3$ | $7-CH_3$ | |

EXAMPLE 27: Preparation of Disodium salt of compound of Example 1

The compound of Example 1 (1-allyl-6,7-dimethoxy-4-hydroxy-7-quinolinone-3-carboxylic acid) (3.0 g) and 800 mg of sodium hydroxide are placed in 75 ml of water and the resulting suspension heated to 50°C until a solution is formed. The water is then evaporated in vacuo and the resulting residue oven dried under high vacuum to obtain 1-allyl-6,7-dimethoxy-4-hydroxy-2-quinolinone-3-carboxylic acid disodium salt, m.p. 320°C with decomposition.

WHAT IS CLAIMED IS:

1. A pharmaceutical composition comprising compounds of formula $I_p$

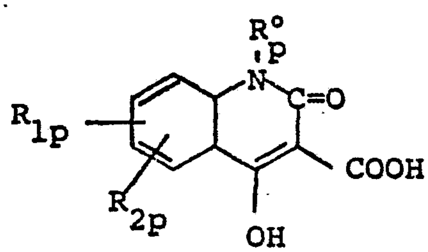

$I_p$

in which $R_p^o$ is hydrogen, alkyl of 1 to 6 carbon atoms, cycloalkyl of 3 to 6 carbon atoms, cycloalkylalkyl in which the cycloalkyl part is of 3 to 6 carbon atoms and the alkyl part is of 1 or 2 carbon atoms, alkenyl or alkynyl of 3 to 6 carbon atoms in which the unsaturation is on other than the alpha carbon atom, or

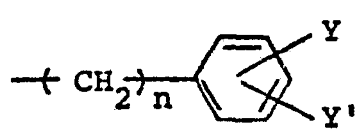

in which n is 0 or 1 and

Y and Y' are independently hydrogen, fluorine, chlorine, bromine, alkyl of 1 to 3

carbon atoms, alkoxy of 1 to 3 carbon atoms, trifluoromethyl or nitro with the proviso that only one of Y and Y' can be from the group consisting of nitro and trifluoromethyl and

$R_{1p}$ and $R_{2p}$ are independently hydrogen, fluorine, chlorine, bromine, alkyl of 1 to 4 carbon atoms, alkoxy of 1 to 4 carbon atoms, nitro or trifluoromethyl, with the proviso that only one of $R_{1p}$ and $R_{2p}$ can be from the group consisting of nitro and trifluoromethyl, or $R_{1p}$ and $R_{2p}$ together form a 6,7-methylenedioxy group,

in free acid or pharmaceutically acceptable mono- or di-base salt form, in association with a pharmaceutically acceptable diluent or carrier.

2. A pharmaceutical composition according to Claim 1 in unit dosage form, each unit dosage comprising 5-400 mg of compound of formula $I_p$.

3. A process for the prepartion of a compound of formula I,

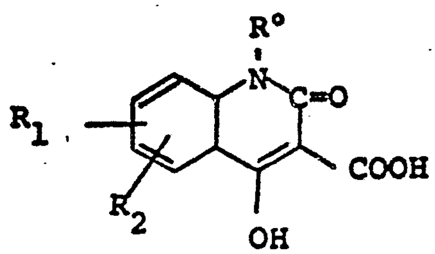

I

in which either

a) R° is cycloalkyl of 3 to 6 carbon atoms, cycloalkylalkyl in which the cycloalkyl part is of 3 to 6 carbon atoms and the alkyl part is of 1 or 2 carbon atoms, alkenyl or alkynyl of 3 to 6 carbon atoms in which the unsaturation is on other than the alpha carbon atom, or

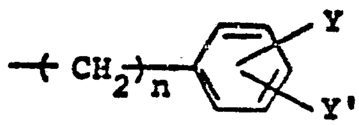

in which n, Y and Y' are as defined above, and $R_1$ and $R_2$ have the same significance as $R_{1p}$ and $R_{2p}$ above, provided that $R_1$ and $R_2$ do not together form a 6,7-methylenedioxy group,

b) R° is hydrogen, $R_1$ is hydrogen, fluorine, chlorine, bromine, alkyl of 1 to 4 carbon atoms or alkoxy of 1 to 4 carbon atoms and $R_2$ is fluorine, chlorine, bromine, alkyl of 1 to 4 carbon atoms, alkoxy of 1 to 4 carbon atoms, nitro or trifluoromethyl,

c) $R^o$ is alkyl of 1 to 6 carbon atoms and $R_1$ and $R_2$ are independently alkoxy of 1 to 4 carbon atoms,

or d) $R^o$ has the same significance as $R^o_p$ above, and $R_1$ and $R_2$ together form a 6,7-methylene-dioxy group,

characterised by hydrolysing a compound of formula II

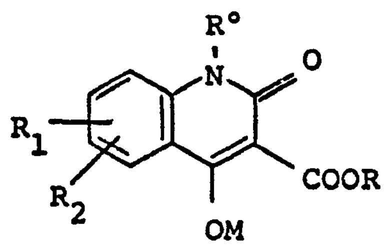

II

in which $R^o$, $R_1$ and $R_2$ are as defined above,

R is alkyl of 1 to 6 carbon atoms,

and M is hydrogen or a pharmaceutically acceptable cation,

provided that when hydrolysis is carried out under acid conditions, R is an acid-labile alkyl group.

4. A compound of formula I, stated in Claim 3, whenever prepared by the process of Claim 3.

5. A compound of formula I, stated in Claim 3.

6. A compound according to Claim 5, in which $R^o$ is hydrogen, alkyl or alkenyl.

7. A compound according to Claim 6, in which $R^o$ is allyl.

8. A compound according to Claim 5, in which $R_1$ and $R_2$ are dialkoxy.

9. A compound according to Claim 8 in which $R_1$ and $R_2$ are 6,7-dimethoxy.

10. 1-Allyl-6,7-dimethoxy-4-hydroxy-2-quinoline-3-carboxylic acid, in free acid, mono- or di-salt form.

European Patent Office

# EUROPEAN SEARCH REPORT

0000153

Application number: EP 78 10 010?

## DOCUMENTS CONSIDERED TO BE RELEVANT

| Category | Citation of document with indication, where appropriate, of relevant passages | Relevant to claim |
|---|---|---|
| | FR - A - 2 228 488 (CIBA-GEIGY)<br>* Claims 1 and 2; page 5, lines 34-38; page 6; page 7, lines 1-6 *<br>-- | 1 |
| P | FR - A - 2 342 067 (SANDOZ)<br>* Claims 1 and 12 *<br>---- | 1,5 |

CLASSIFICATION OF THE APPLICATION (Int. Cl.²)

C 07 D 215/56
A 61 K 31/47

TECHNICAL FIELDS SEARCHED (Int.Cl.²)

C 07 D 215/56
A 61 K 31/47

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 04-09-1978 | ALFARO |